# EUROPEAN PATENT APPLICATION

(11) **EP 4 552 546 A1**
(43) Date of publication of application: **14.05.2025**
(21) Application number: 23860020.9
(22) Date of filing: 14.08.2023
(51) Int. Cl.: A61B 1/00, G02B 23/24

(54) **ENDOSCOPE**

(30) Priority: 31.08.2022 JP 2022138494
(71) Applicant: HOYA CORPORATION, Shinjuku-ku Tokyo 160-8347 (JP)
(72) Inventor: ICHIKURA,Shigeru, Tokyo 160-8347 (JP); WATANABE, Takeharu, Tokyo 160-8347 (JP)
(74) Representative: Lucke, Andreas
(86) International application number: PCT/JP2023/029393
(87) International publication number: WO 2024/048255

(57) **Abstract**

Provided is an endoscope including: a rotary cylindrical portion that is connected with an insertion portion to be inserted into a body lumen and rotates together with the insertion portion; and an operation unit that operates the insertion portion, and the endoscope further includes: a cylindrical handle that has a bottom in which a through hole into which the rotary cylindrical portion is internally fitted is formed and that rotates the rotary cylindrical portion; and a rotation suppression member that is provided in the operation unit, contacts the bottom of the cylindrical handle, and suppresses rotation of the cylindrical handle.

## Description

### Technical Field

The present invention relates to an endoscope including an insertion portion to be inserted into a body lumen.

### Background Art

Conventionally, an endoscope that images an affected part by inserting an insertion portion into a body lumen or the like of a patient and operating an operation unit has been widely used.

For example, Patent Literature 1 discloses an endoscope in which a torque control member is provided between an operation-unit mouthpiece part and an insertion-unit mouthpiece part in a radial direction at an engagement part between the operation-unit mouthpiece part and the insertion-unit mouthpiece part, and frictional resistance by the torque control member is used to prevent an insertion unit from rotating at timing not intended by a user.

### Citation List

### Patent Literature

Patent Literature 1: WO 2012/164978 A1

### Summary of Invention

### Technical Problem

However, in the endoscope of Patent Literature 1, since the torque control member is provided between the operation-unit mouthpiece part and the insertion-unit mouthpiece part in the radial direction, friction between the torque control member and the operation-unit mouthpiece part or the insertion-unit mouthpiece part occurs at the time of assembly work of engaging the operation-unit mouthpiece part and the insertion-unit mouthpiece part. Therefore, such assembly work may be delayed, and eventually, fracture, early deterioration, and the like of the torque control member may occur.

The present invention has been achieved in view of such circumstances, and an object thereof is to provide an endoscope capable of preventing unintended rotation of an insertion portion while facilitating assembly work.

### Solution to Problem

An endoscope according to the present invention is an endoscope including: a rotary cylindrical portion that is connected with an insertion portion to be inserted into a body lumen and rotates together with the insertion portion; and an operation unit that operates the insertion portion, and the endoscope further includes: a cylindrical handle that has a bottom in which a through hole into which the rotary cylindrical portion is internally fitted is formed and that rotates the rotary cylindrical portion; and a rotation suppression member that is provided in the operation unit, contacts the bottom of the cylindrical handle, and suppresses rotation of the cylindrical handle.

In the present invention, the rotation suppression member contacts the bottom of the cylindrical handle to suppress rotation of the cylindrical handle, so that rotation of the rotary cylindrical portion is suppressed through the cylindrical handle. With this configuration, it is possible to prevent the insertion portion connected to the rotary cylindrical portion from rotating regardless of intention of a user.

### Advantageous Effects of Invention

According to the present invention, it is possible to provide an endoscope capable of preventing unintended rotation of an insertion portion while facilitating assembly work.

### Brief Description of Drawings

Fig. 1 is an external view of an endoscope according to a first embodiment of the present invention.
Fig. 2 is an enlarged view enlarging and displaying an operation unit and a bend preventing portion of the endoscope according to the first embodiment.
Fig. 3 is a cross-sectional view of the operation unit and the bend preventing portion along an axial direction.
Fig. 4 is an explanatory view illustrating a configuration of an inner side of a cylindrical handle in Fig. 2.
Fig. 5 is a cross-sectional view representing a state where the cylindrical handle and the bend preventing portion are rotated by a predetermined angle about an axial center.
Fig. 6 is an explanatory view representing a process of assembling the cylindrical handle in the endoscope according to the first embodiment.
Fig. 7 is a partial cross-sectional view illustrating a main configuration of the endoscope according to a modification of the first embodiment.
Fig. 8 is a partial cross-sectional view illustrating a main configuration of an endoscope according to a second embodiment.
Fig. 9 is a partial cross-sectional view illustrating a main configuration of an endoscope according to a third embodiment.

### Description of Embodiments

Hereinafter, an endoscope according to embodiments of the present invention will be described in detail with reference to the drawings.

### (First Embodiment)

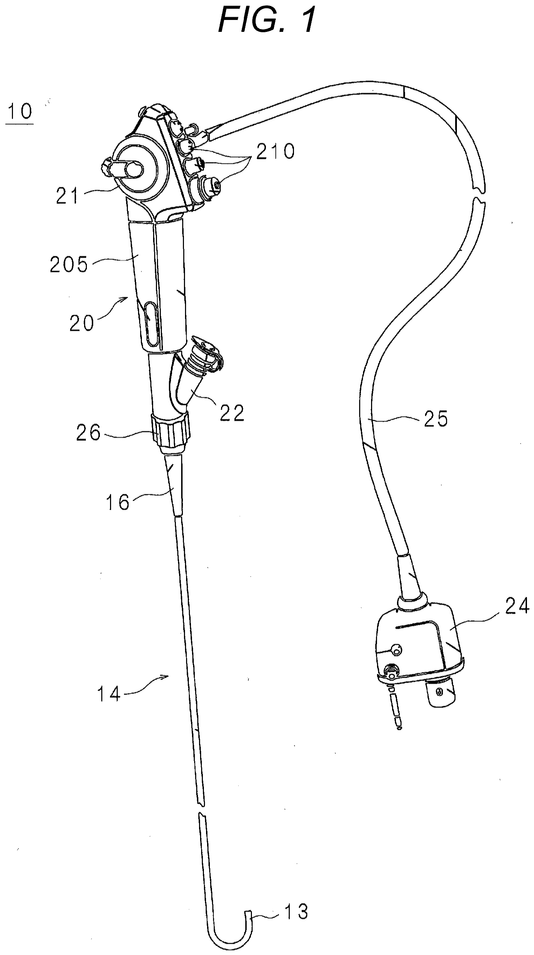

Fig. 1 is an external view of an endoscope 10 according to a first embodiment of the present invention.

The endoscope 10 of the present embodiment includes an insertion portion 14 to be inserted into a body lumen of a subject, an operation unit 20 for operating the insertion portion 14, and a connector unit 24 connected with a processor (not illustrated).

The insertion portion 14 includes a bend preventing portion 16 and is connected with the operation unit 20 through the bend preventing portion 16, and the connector unit 24 is connected with the operation unit 20 through a universal cord 25.

The universal cord 25 has pliability, and includes an electric line that sends, to the connector unit 24, image data (electric signal) from an imaging unit (not illustrated) of an imaging portion 13 to be described later provided at a distal tip of the insertion portion 14.

The operation unit 20 includes a grip portion 205, buttons 210 for receiving an instruction from a user, and a bending operation lever 21 for operating a bending motion of the insertion portion 14.

The grip portion 205 has a substantially cylindrical shape whose diameter gradually decreases toward the bend preventing portion 16, and a channel inlet 22, into which a treatment tool or the like is to be inserted, is provided near a side of the bend preventing portion 16. The grip portion 205 is disposed on the same axial center as the bend preventing portion 16 having a substantially cylindrical shape as described later.

Furthermore, the operation unit 20 (grip portion 205) is provided with a cylindrical handle 26 that rotates the insertion portion 14 at an end portion near the bend preventing portion 16. In a case where a user rotates the cylindrical handle 26, the insertion portion 14 rotates.

The insertion portion 14 includes the imaging portion 13 on a side of the distal tip, and the imaging portion 13 has a cylindrical shape with a small diameter. Furthermore, the bend preventing portion 16 has the substantially cylindrical shape whose diameter gradually decreases toward the insertion portion 14, and suppresses sudden bending of the insertion portion 14.

The imaging portion 13 includes the imaging unit including imaging means such as a charge coupled device (CCD) or a complementary metal oxide semiconductor (CMOS), a circuit board for driving the imaging means, an observation optical system, and the like, and a lens unit (not illustrated) including a series of lens sets. Furthermore, the imaging portion 13 includes an illumination optical system (not illustrated) or the like for irradiating an observation target site in a body lumen with light. An electric signal from the imaging unit of the imaging portion 13 is sent to the processor through the connector unit 24.

As described above, the insertion portion 14 can be bent by an operation of the operation unit 20. That is, the insertion portion 14 is freely bent in two directions in accordance with an operation of the bending operation lever 21.

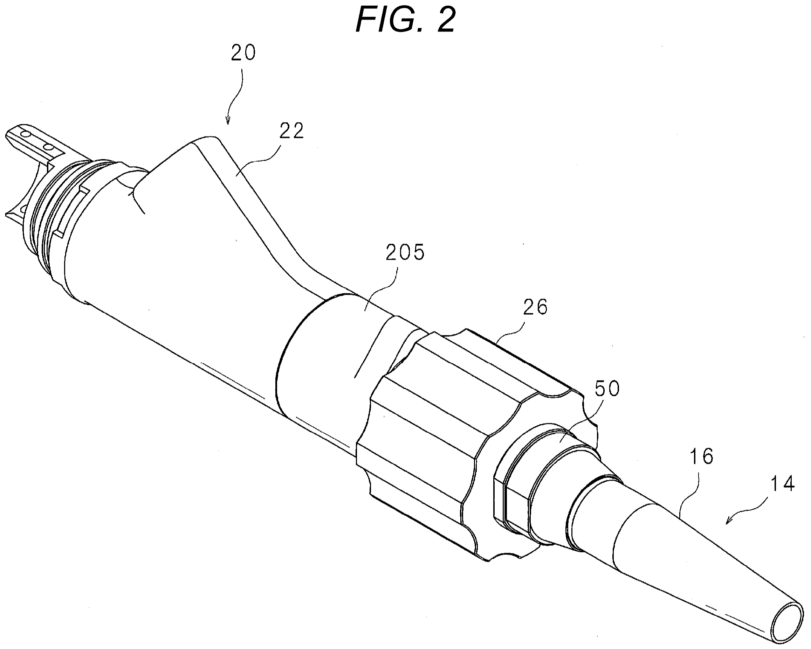

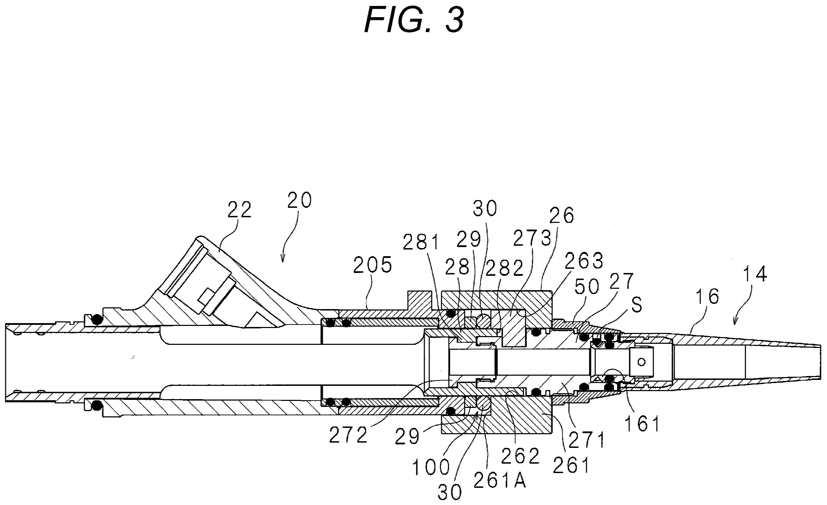

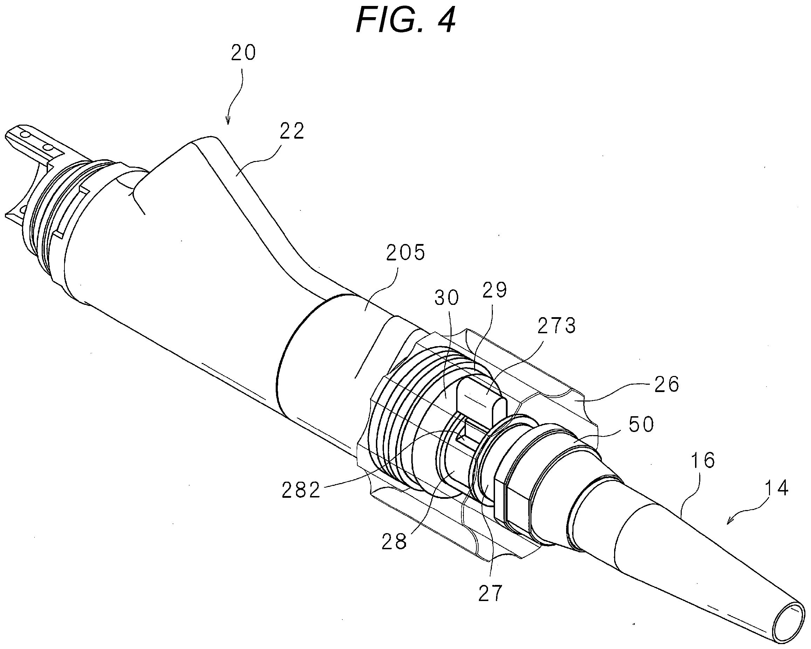

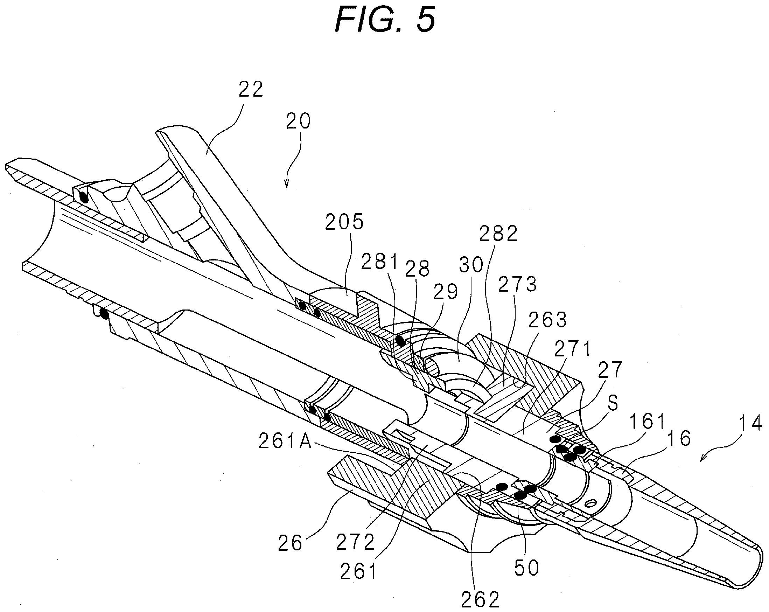

Fig. 2 is an enlarged view enlarging and displaying the operation unit 20 and the bend preventing portion 16 of the endoscope 10 according to the first embodiment, Fig. 3 is a cross-sectional view of the operation unit 20 and the bend preventing portion 16 along an axial direction, Fig. 4 is an explanatory view illustrating a configuration of an inner side of the cylindrical handle 26 in Fig. 2, and Fig. 5 is a cross-sectional view representing a state where the cylindrical handle 26 and the bend preventing portion 16 are rotated by a predetermined angle about an axial center. Fig. 4 illustrates only an outline of the cylindrical handle 26 for convenience. Note that, in Figs. 3 and 5, a cross section of an O-ring is indicated by a black circle.

The operation unit 20 is provided with a rotary cylindrical portion 27 having one end portion connected to one end portion of the bend preventing portion 16 at the end portion on the side of the bend preventing portion 16. Specifically, the operation unit 20 includes a holding cylindrical portion 28, and the rotary cylindrical portion 27 is rotatably held by the holding cylindrical portion 28.

The holding cylindrical portion 28 has a cylindrical shape, and is internally fitted to the one end portion of the grip portion 205 on the side of the bend preventing portion 16. A protrusion 281 is circumferentially provided in an annular shape at a substantially intermediate position in the axial direction on an inner peripheral surface of the holding cylindrical portion 28.

The rotary cylindrical portion 27 has a substantially cylindrical shape, has the other end portion internally fitted to the holding cylindrical portion 28, and has the one end portion externally fitted to the one end portion of the bend preventing portion 16. A groove is formed in a circumferential direction on an outer peripheral surface of the other end portion of the rotary cylindrical portion 27, and such a groove is engaged with the protrusion 281 of the holding cylindrical portion 28. With this configuration, movement of the rotary cylindrical portion 27 in the axial direction is restricted.

The rotary cylindrical portion 27 includes a first cylindrical portion 271 on a side of the one end portion and a second cylindrical portion 272 on a side of the other end portion.

The first cylindrical portion 271 and the second cylindrical portion 272 are coaxially disposed and fitted.

One end portion of the first cylindrical portion 271 has an enlarged inner diameter, and is externally fitted to a diameter-reduced portion 161 provided at the one end portion of the bend preventing portion 16. An O-ring is interposed between the one end portion of the first cylindrical portion 271 and the diameter-reduced portion 161 of the bend preventing portion 16. Furthermore, a screw hole penetrating in a radial direction is formed at the one end portion of the first cylindrical portion 271, and a recess is formed on an outer peripheral surface of the diameter-reduced portion 161 corresponding to such a screw hole. When a so-called setscrew S is inserted through the screw hole and an end portion of the setscrew S is engaged with the recess of the diameter-reduced portion 161, the bend preventing portion 16 is integrated with the first cylindrical portion 271 (rotary cylindrical portion 27) and rotates together with the rotary cylindrical portion 27 as described above (see Fig. 5).

Furthermore, the other end portion of the first cylindrical portion 271 has an enlarged inner diameter on a side of the second cylindrical portion 272, and is externally fitted to one end portion of the second cylindrical portion 272.

Furthermore, the first cylindrical portion 271 of the rotary cylindrical portion 27 is provided with a connection portion 273 that connects the rotary cylindrical portion 27 with the cylindrical handle 26. The connection portion 273 protrudes outward in the radial direction at one portion on an outer peripheral surface of the first cylindrical portion 271 (see Fig. 4), and has a distal tip bent toward the side of the second cylindrical portion 272. A distal tip surface of the connection portion 273 in the axial direction of the rotary cylindrical portion 27 is flush with a bottom surface 261A of a bottom 261 to be described later of the cylindrical handle 26.

Note that, hereinafter, the first cylindrical portion 271 or the second cylindrical portion 272 is also referred to as the rotary cylindrical portion 27.

The holding cylindrical portion 28 is formed with a cutout 282 at an end portion on the side of the bend preventing portion 16. The cutout 282 is formed in a part of the end portion along the circumferential direction. The connection portion 273 is disposed inside the cutout 282, and an edge of the cutout 282 in the axial direction of the holding cylindrical portion 28 is positioned between the outer peripheral surface of the first cylindrical portion 271 and the bent distal tip of the connection portion 273. Therefore, when the rotary cylindrical portion 27 rotates about the axial center, the connection portion 273 also moves in the cutout 282, but a movement range of the connection portion 273 is restricted to between both edges of the cutout 282 in the circumferential direction. That is, a rotation range of the rotary cylindrical portion 27 is restricted within a predetermined angle.

The cylindrical handle 26 has a bottomed cylindrical shape, and has one end portion in the axial direction externally fitted to the one end portion of the grip portion 205. An O-ring is interposed between the one end portion of the cylindrical handle 26 and the one end portion of the grip portion 205. Furthermore, the cylindrical handle 26 has the bottom 261 at the other end portion in the axial direction, and the rotary cylindrical portion 27 penetrates the bottom 261 from the inside to the outside. That is, a through hole 262 is formed in a central portion of the bottom 261, and the rotary cylindrical portion 27 (first cylindrical portion 271) is internally fitted to the through hole 262. An O-ring is interposed between the first cylindrical portion 271 and an inner peripheral surface of the through hole 262.

Furthermore, in the bottom 261, at a position corresponding to the connection portion 273, an engagement recess 263 having a shape following the connection portion 273 and engaged with the connection portion 273 is formed. Therefore, in a case where the cylindrical handle 26 rotates, the rotary cylindrical portion 27 and the bend preventing portion 16 (insertion portion 14) also rotate together.

A fixing cylinder 50 for fixing the cylindrical handle 26 is provided at the one end portion of the first cylindrical portion 271 of the rotary cylindrical portion 27 outside the bottom 261 of the cylindrical handle 26. The fixing cylinder 50 has a substantially cylindrical shape, and is externally fitted to the one end portion of the first cylindrical portion 271. An O-ring is interposed between the fixing cylinder 50 and the one end portion of the first cylindrical portion 271.

Additionally, a positioning nut 29 (contact portion) that determines a positional relationship between the grip portion 205 and the holding cylindrical portion 28 is provided outside the holding cylindrical portion 28. The positioning nut 29 has an annular body shape having a rectangular cross section, and is screwed with a thread (not illustrated) formed on an outer peripheral surface of the holding cylindrical portion 28. At this time, one end in the axial direction of the positioning nut 29 is in contact with the one end portion of the grip portion 205.

A rotation suppression member 30 is disposed outside the holding cylindrical portion 28 and between the bottom 261 of the cylindrical handle 26 and the positioning nut 29. The rotation suppression member 30 is externally fitted to the holding cylindrical portion 28. For example, the rotation suppression member 30 has a ring shape having a circular cross section (see Fig. 4 or Fig. 5), and includes a material having excellent frictional resistance, such as rubber or silicon.

The rotation suppression member 30 contacts the bottom 261 of the cylindrical handle 26 to suppress rotation of the cylindrical handle 26. That is, the rotation suppression member 30 contacts the other end in the axial direction of the positioning nut 29, contacts the outer peripheral surface of the holding cylindrical portion 28, and also contacts the bottom surface 261A of the cylindrical handle 26. Therefore, rotation of the cylindrical handle 26 is suppressed by frictional resistance generated between the rotation suppression member 30 and the bottom surface 261A of the cylindrical handle 26. Accordingly, rotation of the insertion portion 14 connected to the cylindrical handle 26 is also suppressed.

On the other hand, the rotation suppression member 30 is disposed away from an inner surface of the cylindrical handle 26. That is, a gap 100 is formed between the rotation suppression member 30 and the cylindrical handle 26 in the radial direction.

The endoscope 10 according to the first embodiment having the above-described configuration can prevent the insertion portion 14 from rotating at timing not intended by the user in advance.

As described above, the rotation suppression member 30 contacts the other end in the axial direction of the positioning nut 29, contacts the outer peripheral surface of the holding cylindrical portion 28, and also contacts the bottom surface 261A of the cylindrical handle 26. At this time, rotation of the cylindrical handle 26 is suppressed by frictional resistance generated between the other end in the axial direction of the positioning nut 29 and the rotation suppression member 30, between the outer peripheral surface of the holding cylindrical portion 28 and the rotation suppression member 30, and between the bottom surface 261A of the cylindrical handle 26 and the rotation suppression member 30. That is, unless an external force is applied, that is, unless a user who desires rotation of the insertion portion 14 intentionally rotates the cylindrical handle 26, the rotation of the insertion portion 14 is suppressed.

Furthermore, in the endoscope 10 according to the first embodiment, the gap 100 is formed between the rotation suppression member 30 and the cylindrical handle 26 in the radial direction, and as described above, the rotation suppression member 30 contacts the cylindrical handle 26 (bottom surface 261A) only in the axial direction. Therefore, damage to the rotation suppression member 30 at the time of assembly can be prevented. The details will be described below.

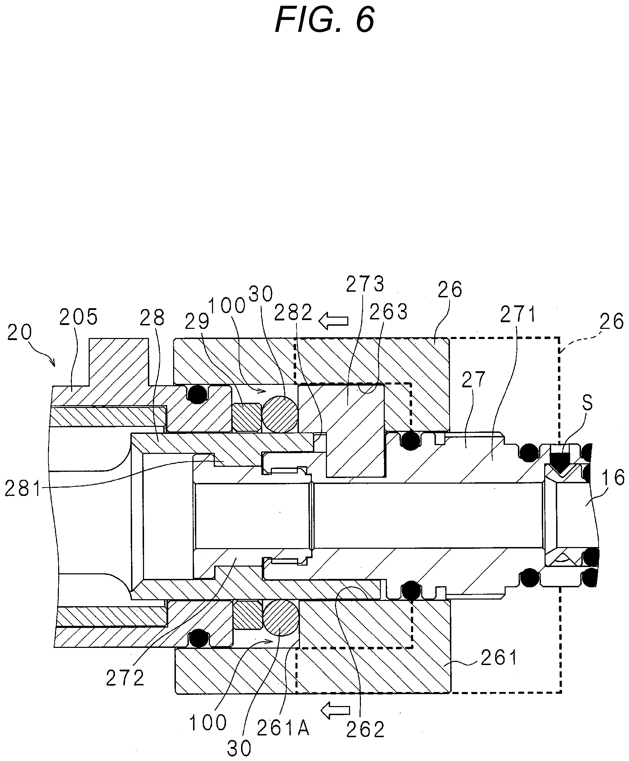

Fig. 6 is an explanatory view representing a process of assembling the cylindrical handle 26 in the endoscope 10 according to the first embodiment. In Fig. 6, a state where the assembly is completed is illustrated, and a broken line indicates the cylindrical handle 26 in the middle of being assembled. Furthermore, in Fig. 6, a state before the fixing cylinder 50 for fixing the cylindrical handle 26 is assembled is illustrated.

The cylindrical handle 26 is fitted to the rotary cylindrical portion 27 from the side of the bend preventing portion 16, and is externally fitted to the rotary cylindrical portion 27 (first cylindrical portion 271) as described above. At this time, as illustrated in Fig. 6, the cylindrical handle 26 moves in an arrow direction of Fig. 6 on the outer peripheral surface of the rotary cylindrical portion 27. However, since the gap 100 is formed between the rotation suppression member 30 and the cylindrical handle 26, the inner surface of the cylindrical handle 26 does not contact the rotation suppression member 30, and the rotation suppression member 30 is not rubbed. Therefore, workability of assembly work can be enhanced, and damage to the rotation suppression member 30 at the time of assembly can be prevented.

### (Modification)

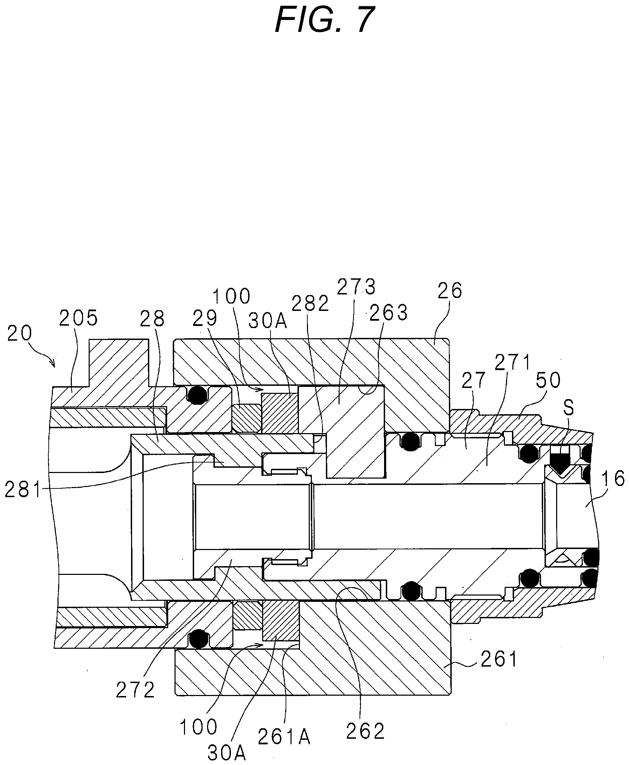

Fig. 7 is a partial cross-sectional view illustrating a main configuration of the endoscope 10 according to a modification of the first embodiment. For convenience, in Fig. 7, vicinity of the rotary cylindrical portion 27 is illustrated in an enlarged manner. The endoscope 10 according to the modification includes a rotation suppression member 30A, and a shape of the rotation suppression member 30A is different from that of the rotation suppression member 30 of the first embodiment.

The rotation suppression member 30A is disposed outside the holding cylindrical portion 28 and between the bottom 261 of the cylindrical handle 26 and the positioning nut 29. The rotation suppression member 30A is externally fitted to the holding cylindrical portion 28. For example, the rotation suppression member 30A has an annular body shape having a rectangular cross section, and includes a material having excellent frictional resistance, such as rubber or silicon.

The rotation suppression member 30A contacts the other end in the axial direction of the positioning nut 29, contacts the outer peripheral surface of the holding cylindrical portion 28, and also contacts the bottom surface 261A of the cylindrical handle 26. Therefore, frictional resistance generated between the rotation suppression member 30A and the bottom surface 261A of the cylindrical handle 26 suppresses rotation of the cylindrical handle 26, and also suppresses rotation of the insertion portion 14 connected with the cylindrical handle 26.

On the other hand, as in the first embodiment, the gap 100 is formed between the rotation suppression member 30A and the cylindrical handle 26 in the radial direction.

With the above configuration, the rotation suppression member 30A contacts the other end in the axial direction of the positioning nut 29, contacts the outer peripheral surface of the holding cylindrical portion 28, and also contacts the bottom surface 261A of the cylindrical handle 26. Since the rotation suppression member 30A has the rectangular cross section, a contact area with the other end in the axial direction of the positioning nut 29, a contact area with the outer peripheral surface of the holding cylindrical portion 28, and a contact area with the bottom surface 261A of the cylindrical handle 26 can be further expanded. Therefore, frictional resistance generated between the other end in the axial direction of the positioning nut 29 and the rotation suppression member 30A, between the outer peripheral surface of the holding cylindrical portion 28 and the rotation suppression member 30A, and between the bottom surface 261A of the cylindrical handle 26 and the rotation suppression member 30A increases. Therefore, it is possible to more effectively prevent the insertion portion 14 from rotating at timing not intended by a user.

Furthermore, in the endoscope 10 according to the modification, the gap 100 is formed between the rotation suppression member 30A and the cylindrical handle 26 in the radial direction, and the rotation suppression member 30A contacts the cylindrical handle 26 (bottom surface 261A) only in the axial direction. Therefore, workability of assembly work can be enhanced, and it is possible to prevent the rotation suppression member 30A from being damaged by being rubbed by contacting the cylindrical handle 26 at the time of assembly.

### (Second Embodiment)

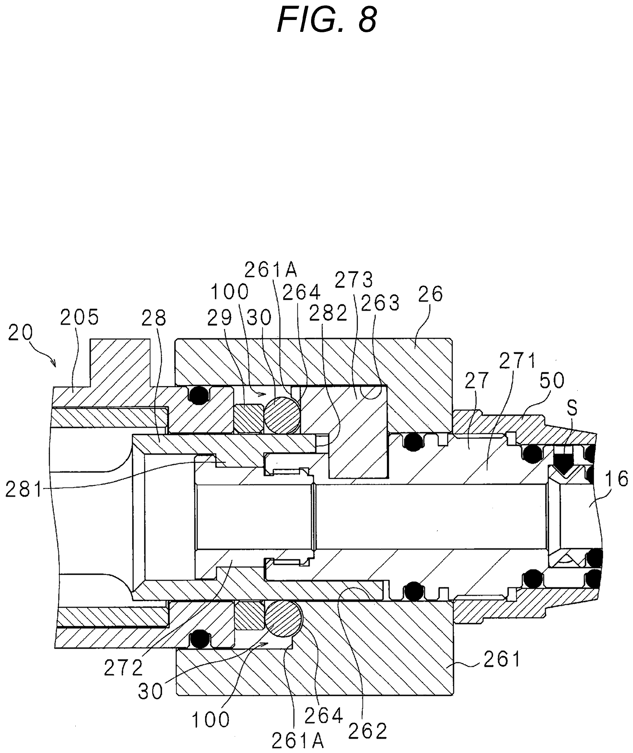

Fig. 8 is a partial cross-sectional view illustrating a main configuration of an endoscope 10 according to a second embodiment. For convenience, in Fig. 8, vicinity of a rotary cylindrical portion 27 is illustrated in an enlarged manner.

In the endoscope 10 according to the second embodiment, as in the first embodiment, a rotation suppression member 30 is disposed outside a holding cylindrical portion 28 and between a bottom 261 of a cylindrical handle 26 and a positioning nut 29. The rotation suppression member 30 is externally fitted to the holding cylindrical portion 28. For example, the rotation suppression member 30 has a ring shape having a circular cross section, and includes a material having excellent frictional resistance, such as rubber or silicon.

The rotation suppression member 30 contacts the other end in an axial direction of the positioning nut 29, contacts an outer peripheral surface of the holding cylindrical portion 28, and also contacts a bottom surface 261A of the cylindrical handle 26.

More specifically, on the bottom surface 261A of the cylindrical handle 26, a groove 264 is formed at a position facing the rotation suppression member 30 in the axial direction. That is, the groove 264 is formed in a circular shape in a circumferential direction of the holding cylindrical portion 28. The groove 264 has a curved surface corresponding to the rotation suppression member 30 on an inner side, and the rotation suppression member 30 is partially housed in the groove 264.

At this time, frictional resistance generated between the rotation suppression member 30 and the bottom surface 261A of the cylindrical handle 26 suppresses rotation of the cylindrical handle 26, and also suppresses rotation of an insertion portion 14 connected with the cylindrical handle 26.

On the other hand, as in the first embodiment, a gap 100 is formed between the rotation suppression member 30 and the cylindrical handle 26 in a radial direction.

With the above configuration, the rotation suppression member 30 contacts the other end in the axial direction of the positioning nut 29, contacts the outer peripheral surface of the holding cylindrical portion 28, and also contacts the bottom surface 261A of the cylindrical handle 26.

Moreover, since the groove 264 is formed in the bottom surface 261A of the cylindrical handle 26 and the rotation suppression member 30 is partially housed in the groove 264, a large contact area between the rotation suppression member 30 and the bottom surface 261A of the cylindrical handle 26 can be secured. Therefore, frictional resistance generated between the bottom surface 261A of the cylindrical handle 26 and the rotation suppression member 30 increases. Therefore, it is possible to more effectively prevent the insertion portion 14 from rotating at timing not intended by a user.

Furthermore, as described above, since the rotation suppression member 30 is partially housed in the groove 264, a position of the rotation suppression member 30 is determined, and deviation of the position of the rotation suppression member 30 from a normal position can be suppressed.

Moreover, also in the endoscope 10 according to the second embodiment, the gap 100 is formed between the rotation suppression member 30 and the cylindrical handle 26 in the radial direction, and the rotation suppression member 30 contacts the cylindrical handle 26 (bottom surface 261A) only in the axial direction. Therefore, workability of assembly work can be enhanced, and it is possible to prevent the rotation suppression member 30 from being damaged by being rubbed by contacting the cylindrical handle 26 at the time of assembly.

Other configurations in the endoscope 10 of the second embodiment are the same as those of the endoscope 10 in the first embodiment, and portions similar to those of the first embodiment are denoted by the same reference signs, and detailed descriptions will be omitted.

### (Third Embodiment)

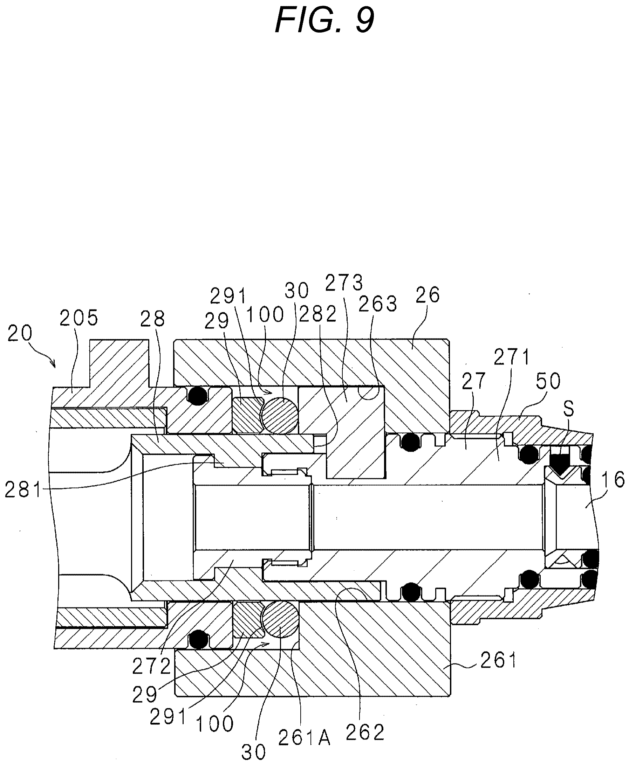

Fig. 9 is a partial cross-sectional view illustrating a main configuration of an endoscope 10 according to a third embodiment. For convenience, in Fig. 9, vicinity of a rotary cylindrical portion 27 is illustrated in an enlarged manner.

In the endoscope 10 according to the third embodiment, as in the first embodiment, a rotation suppression member 30 is disposed outside a holding cylindrical portion 28 and between a bottom 261 of a cylindrical handle 26 and a positioning nut 29. The rotation suppression member 30 is externally fitted to the holding cylindrical portion 28. For example, the rotation suppression member 30 has a ring shape having a circular cross section, and includes a material having excellent frictional resistance, such as rubber or silicon.

The rotation suppression member 30 contacts the other end in an axial direction of the positioning nut 29, contacts an outer peripheral surface of the holding cylindrical portion 28, and also contacts a bottom surface 261A of the cylindrical handle 26.

More specifically, at the other end in the axial direction of the positioning nut 29, a groove 291 is formed at a position facing the rotation suppression member 30 in the axial direction. That is, the groove 291 is formed in a circular shape along the other end surface in the axial direction of the positioning nut 29. The groove 291 has a curved surface corresponding to the rotation suppression member 30 on an inner side, and the rotation suppression member 30 is partially housed in the groove 291.

At this time, frictional resistance generated between the rotation suppression member 30 and the bottom surface 261A of the cylindrical handle 26 suppresses rotation of the cylindrical handle 26, and also suppresses rotation of an insertion portion 14 connected with the cylindrical handle 26.

On the other hand, as in the first embodiment, a gap 100 is formed between the rotation suppression member 30 and the cylindrical handle 26 in a radial direction.

With the above configuration, the rotation suppression member 30 contacts the other end in the axial direction of the positioning nut 29, contacts the outer peripheral surface of the holding cylindrical portion 28, and also contacts the bottom surface 261A of the cylindrical handle 26.

Moreover, since the groove 291 is formed at the other end in the axial direction of the positioning nut 29 and the rotation suppression member 30 is partially housed in the groove 291, a contact area between the rotation suppression member 30 and the other end in the axial direction of the positioning nut 29 can be increased. Therefore, frictional resistance generated between the other end in the axial direction of the positioning nut 29 and the rotation suppression member 30 increases, and a suppression force for suppressing movement of the rotation suppression member 30 increases. Therefore, it is possible to improve a rotation prevention effect of the cylindrical handle 26 due to frictional resistance with the rotation suppression member 30, and it is possible to more effectively prevent the insertion portion 14 from rotating at timing not intended by a user.

Furthermore, as described above, since the rotation suppression member 30 is partially housed in the groove 291, a position of the rotation suppression member 30 is determined, and deviation of the position of the rotation suppression member 30 from a normal position can be suppressed.

Moreover, also in the endoscope 10 according to the third embodiment, the gap 100 is formed between the rotation suppression member 30 and the cylindrical handle 26 in the radial direction, and the rotation suppression member 30 contacts the cylindrical handle 26 (bottom surface 261A) only in the axial direction. Therefore, workability of assembly work can be enhanced, and it is possible to prevent the rotation suppression member 30 from being damaged by being rubbed by contacting the cylindrical handle 26 at the time of assembly.

Other configurations in the endoscope 10 of the third embodiment are the same as those of the endoscope 10 in the first embodiment, and portions similar to those of the first embodiment are denoted by the same reference signs, and detailed descriptions will be omitted.

Additionally, the rotation suppression member 30 may be disposed away from the outer peripheral surface of the holding cylindrical portion 28. That is, a gap may be formed between the rotation suppression member 30 and the outer peripheral surface of the holding cylindrical portion 28 in the radial direction. At this time, it is possible to further prevent the rotation suppression member 30 from being damaged by being rubbed by contacting the holding cylindrical portion 28.

Technical features (constitutional requirements) that have been described in the first to third embodiments can be combined with one another, and a new technical feature can be formed with the combination.

The embodiments disclosed this time should be considered to be exemplary in all respects and not to be restrictive. The scope of the present invention is indicated by the scope of claims, not the above-described significance, and is intended to include all modifications within the significance and scope equivalent to the claims.

### Reference Signs List

- 10: Endoscope
- 13: Imaging portion
- 14: Insertion portion
- 18: Connection portion
- 20: Operation unit
- 26: Cylindrical handle
- 27: Rotary cylindrical portion
- 28: Holding cylindrical portion
- 29: Positioning nut
- 30: Rotation suppression member
- 30A: Rotation suppression member
- 100: Gap
- 261: Bottom
- 261A: Bottom surface
- 262: Through hole
- 264: Groove
- 291: Groove

## Claims

1. An endoscope comprising: a rotary cylindrical portion that is connected with an insertion portion to be inserted into a body lumen and rotates together with the insertion portion; and an operation unit that operates the insertion portion, the endoscope further comprising:
a cylindrical handle that has a bottom in which a through hole into which the rotary cylindrical portion is internally fitted is formed and that rotates the rotary cylindrical portion; and
a rotation suppression member that is provided in the operation unit, contacts the bottom of the cylindrical handle, and suppresses rotation of the cylindrical handle.

2. The endoscope according to claim 1, further comprising
a holding cylindrical portion that is externally fitted to the rotary cylindrical portion and rotatably holds the rotary cylindrical portion,
wherein the rotation suppression member is circumferentially provided outside the holding cylindrical portion.

3. The endoscope according to claim 1 or 2, wherein a gap is formed between the rotation suppression member and an inner surface of the cylindrical handle.

4. The endoscope according to claim 1 or 2, wherein the rotation suppression member has a ring shape.

5. The endoscope according to claim 1 or 2, wherein the rotation suppression member is an annular body shape having a rectangular cross section.

6. The endoscope according to claim 1 or 2, further comprising a groove formed in the bottom of the cylindrical handle and having a shape corresponding to the rotation suppression member.

7. The endoscope according to claim 1 or 2, further comprising
a contact portion that contacts the rotation suppression member on a side opposite to the bottom in an axial direction of the rotary cylindrical portion,
wherein a groove having a shape corresponding to the rotation suppression member is formed in the contact portion.
